(19) Europäisches Patentamt / European Patent Office / Office européen des brevets

(11) **EP 4 521 413 A1**

(12) **EUROPEAN PATENT APPLICATION**
published in accordance with Art. 153(4) EPC

(43) Date of publication:
**12.03.2025 Bulletin 2025/11**

(21) Application number: **24781372.8**

(22) Date of filing: **29.03.2024**

(51) International Patent Classification (IPC):
*G16C 20/70* (2019.01)    *G16C 20/50* (2019.01)
*G16C 20/40* (2019.01)    *G16C 20/20* (2019.01)
*G16C 20/30* (2019.01)    *G16C 20/80* (2019.01)
*G16C 20/90* (2019.01)

(52) Cooperative Patent Classification (CPC):
**G16C 20/20; G16C 20/30; G16C 20/40;
G16C 20/50; G16C 20/70; G16C 20/80;
G16C 20/90**

(86) International application number:
**PCT/KR2024/095643**

(87) International publication number:
**WO 2024/205384 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.03.2023 KR 20230042882**

(71) Applicant: **LG Chem, Ltd.**
**Yeongdeungpo-gu**
**Seoul 07336 (KR)**

(72) Inventors:
• **JO, Seong In**
  **Daejeon 34122 (KR)**
• **LEE, Kyu Hwang**
  **Daejeon 34122 (KR)**
• **BAE, Yong Jin**
  **Daejeon 34122 (KR)**
• **YOU, Hyun Sik**
  **Daejeon 34122 (KR)**
• **PARK, Sae Bo Mi**
  **Daejeon 34122 (KR)**
• **YEU, Yun Ku**
  **Daejeon 34122 (KR)**

(74) Representative: **Plasseraud IP**
**104 Rue de Richelieu**
**CS92104**
**75080 Paris Cedex 02 (FR)**

(54) **METHOD AND SYSTEM FOR ACQUIRING MOLECULAR STRUCTURE OF PARENT MOLECULE FROM FRAGMENT IONS FROM LC-MS/MS**

(57) Disclosed herein are a method and a system of acquiring mass spectrum peak data of fragment ions from mass spectra for fragment ions generated in an LC-MS/MS measuring instrument, acquiring a molecular structure of the fragment ions and fragment ion graph data by using the acquired data, and deriving a candidate molecular structure of a target substance by assembling fragment ion graphs.

**Fig. 1**

EP 4 521 413 A1

**Description**

## TECHNICAL FIELD

The present invention relates to a technology for identifying and analyzing a molecular structure of a parent molecule (original compound) based on information on fragment ions generated during liquid chromatography-tandem mass spectrometry (LC-MS/MS) analysis.

## BACKGROUND ART

When a structure of a material is analyzed by liquid chromatography-tandem mass spectrometry (LC-MS/MS) analysis, strong energy is applied to sample molecules to ionize and fragment the molecules, and then the mass is measured based on the speed of each fragment ion. In this case, the structure of a parent molecule may be estimated based on the elemental composition and detection intensity of each fragment ion and the elemental composition of the parent molecule.

However, in order to implement the estimation, since the structure of the parent molecule has to be estimated based on the elemental composition and detection intensity of each fragment ion and the elemental composition of the parent molecule, it is necessary to be able to estimate the structure of individual fragment ions and then draw the structure of the parent molecule including all the structures of the fragment ions as partial structures.

However, usually, it is not possible to accurately estimate the structure of a fragment ion using only the information provided by the MS/MS spectrum, and each fragment ion has multiple possible candidate structures. Therefore, in order to estimate the structure of the parent molecule using the structures of the fragment ion, both structural combinations of respective fragment ions and the number of cases of assembling thereof have to be explored, which leads to a problem that the computation time exponentially increases with respect to the average number of nodes in a graph to be assembled. For example, the number of subgraphs in a graph of size N is $2^N$, and since subgraph isomorphism is also an NP-complete problem, it is not possible to solve the combination problem of all subgraphs within polynomial time.

Prior arts related to the present invention are as follows.

JP 2019-100891 A (June 24, 2019)

High-Resolution Liquid Chromatography Tandem Mass Spectrometry Enables Large Scale Molecular Characterization of Dissolved Organic Matter, Danial Petras, et. Al, METHODS, 12 December 2017 doi:10.3389/fmars.2017.00405

## DISCLOSURE OF THE INVENTION

### TECHNICAL PROBLEM

The present invention is intended to provide a method and a system of estimating a molecular structure of a target substance using liquid chromatography-tandem mass spectrometry (LC-MS/MS).

In order to solve the aforementioned problems in the related art, the present invention is intended to provide an algorithm capable of solving a problem of binding fragment ions calculated for the target substance within polynomial time and a system to which the algorithm is applied, thereby making it possible to estimate the molecular structure of the target substance.

### TECHNICAL SOLUTION

In order to solve the aforementioned problems, according to the present invention, there is provided a method of acquiring a molecular structure of a target substance, the method including a fragment ion candidate structure derivation step of deriving candidate structures of fragment ions by inputting mass spectra of fragment ions acquired from LC-MS/MS analysis of the target substance into a predetermined spectrum-molecular structure database, a fragment ion graphing step of converting the candidate structures of the derived fragment ions into fragment ion graph data, a fragment ion graph assembly step of assembling fragment ion candidate structures by using the fragment ion graph data to acquire candidate molecular structures of the target substance, and a target substance molecular structure determination step of determining a target substance molecular structure from the candidate molecular structures of the target substance in the fragment ion graph assembly step.

To this end, the present invention applies a method of expressing candidate structures of the fragment ion as fragment ion graphs by expressing invariable atomic groups included in the fragment ion and the linking atoms linked to the invariable atomic groups as nodes and expressing the information about the atomic group or linking atom represented by each node as attribute information about each node.

In assembling the fragment ion graphs, the present invention provides a method of a tree node assignment step (S31) of assigning fragment ion graphs corresponding to each peak of the mass spectrum to a tree node variable in order to perform a tree search operation, a visit node selection step (S32) of sequentially selecting and visiting tree nodes of a first depth, excluding tree nodes set as abort nodes, a child tree node creation step (S33) of setting the visited tree node as a parent node and linking all tree nodes corresponding to a following peak as child nodes, an assembly intermediate creation step (S34) of creating assembly intermediates in the child nodes by sequentially assembling the fragment ion graphs assigned to the child nodes with assembly intermediate graphs assigned to intermediate node variables of the parent node, assigning an assembly intermediate to an assembly intermediate node variable corresponding to a corresponding child node, and setting the corresponding child node as the abort node when a predetermined assembly intermediate binding condition is not satisfied, and acquiring, as candidate molecular structures of the target substance, final assembly intermediates created by repeating the visit node selection step to the assembly intermediate creation step until there are no more child tree nodes to be created.

In addition, as a method of creating an assembly intermediate by assembling fragment ion graphs, the present invention provides a graph assembly method including an assembly target fragment ion selection step of selecting the assembly intermediate graph of the parent node and the fragment ion graph of the child node as target fragment ion graph data to be assembled and an input assembly fragment ion graph to be assembled, respectively, a subgraph data extraction step of extracting all subgraphs from the input fragment ion graph data, and a subgraph combination step of sequentially combining all the subgraphs into the target fragment ion graph.

In addition, according to the present invention, there is provided a system of acquiring a molecular structure of a target substance, the system including an LC-MS/MS measuring unit configured to separate the target substance into fragment ions and generate mass spectra for the fragment ions, a spectrum preprocessing module configured to acquire mass spectrum peak data of fragment ions from the mass spectra of the fragment ions, acquire the molecular structures of fragment ions from a spectrum-molecular structure database using the mass spectrum peak data, and perform fragment ion graphing on the molecular structures of fragment ions to produce fragment ion graph data, a graph assembly module configured to derive candidate molecular structures of the target substance by receiving the fragment ion graph data and assembling fragment ion graphs, and a spectrum-molecular structure database configured to output molecular structures of the fragment ions by receiving mass spectral peak data on the fragment ions.

## ADVANTAGEOUS EFFECTS

According to the present invention, it is possible to estimate a molecular structure of a target substance by applying a method of deriving a fragment ion graph corresponding to each peak of a fragment ion mass spectrum and assembling fragment ion graphs through a tree search technique.

## BRIEF DESCRIPTION OF THE DRAWINGS

The following drawings attached to the specification illustrate preferred embodiments of the present invention by example, and serve to enable technical aspects of the present invention to be further understood together with detailed description of the invention given above, and therefore the present invention should not be interpreted only with matters in such drawings.

FIG. 1 is a block diagram of a system of acquiring a molecular structure of a parent molecule from fragment ions according to the present invention.

FIG. 2 is an example of mass spectra acquired in the present invention.

FIG. 3 is a flowchart showing a procedure for acquiring the molecular structure of the parent molecule from the fragment ions of the present invention.

FIG. 4 is a diagram for describing graphing of fragment ions according to the present invention.

FIG. 5 is a diagram for describing a process of assembling fragment ion graphs corresponding to mass spectrum peaks according to the present invention.

FIG. 6 is a flowchart showing the process of assembling fragment ion graphs corresponding to mass spectrum peaks according to the present invention.

FIG. 7 is a diagram showing a process of combining fragment ion graphs according to a search tree procedure according to the present invention.

FIG. 8 is a diagram showing an example of assembling a subgraph into an assembly target graph according to the present invention.

FIG. 9 is a flowchart showing a procedure for assembling a subgraph into an assembly target graph according to the present invention.

FIG. 10 is a diagram showing a process of assembling by applying subgraph isomorphism.

FIG. 11 is an example of pseudocode for implementing a subgraph assembly algorithm according to the present invention.

FIG. 12 is an example of deriving a structure of a parent molecule from graph assembly results according to the present invention.

## MODE FOR CARRYING OUT THE INVENTION

For such a parent molecule structure estimation, it is necessary to estimate the structure of individual fragment ions and then draw the structure of the parent molecule including all of them as partial structures. However, since it is not possible to accurately estimate the structure of a fragment ion using only information provided by the MS/MS spectrum, each fragment ion corresponds to multiple possible candidate structures, and in order to estimate the structure of the parent molecule using the candidate structures, both the structural combinations of respective candidate fragment ions and the number of cases of assembling them have to be explored.

To this end, the present invention allows each fragment structure to overlap each other and assembles it, but estimates the structure of the parent molecule through an algorithm that extracts the number of all cases in which a molecule from which all pieces are assembled is identical to the elemental composition of the parent molecule.

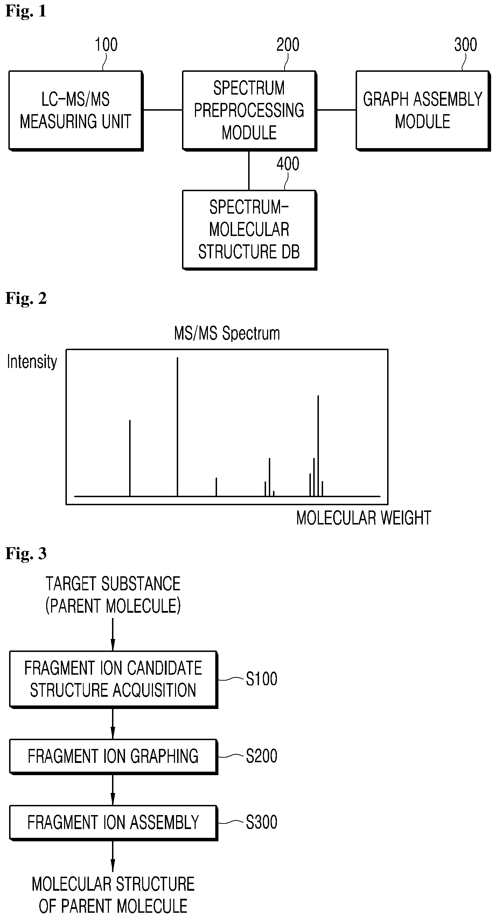

Hereinafter, referring to FIGS. 1 to 12, a method and a system of acquiring a molecular structure of a target substance (parent molecule) from fragment ions generated during liquid chromatography-tandem mass spectrometry (LC-MS/MS LC-MS/MS) analysis of the target substance, according to the present invention, will be described.

1. Parent molecule molecular structure acquisition system

As shown in FIG. 1, a system of the present invention includes an LC-MS/MS measuring unit 100, a spectrum preprocessing module 200, a graph assembly module 300, and a spectrum-molecular structure database (DB) 400.

The LC-MS/MS measuring unit 100 includes a typical LC-MS/MS measuring instrument (liquid chromatography-tandem mass spectrometry (LC-MS/MS)). The device separates a target substance (parent molecule) into fragment ions and generates mass spectra for the fragment ions.

FIG. 2 shows an example of mass spectra generated from fragment ions. The mass spectra for fragment ions may be acquired as a graph constituted by a mass axis (molecular weight) and an ionic intensity axis that represent the mass of each fragment ion in mass-charge ratio (m/z), and when a specific mass (m/z) in the mass spectrum of a fragment ion is found in the graph, the molecular weight of the fragment ion may be calculated. In this case, since the quantity of charge of the fragment ion is usually 1, the mass and molecular weight of the fragment ion may be said to be almost the same, and therefore, the m/z value of the fragment ion may be used as the molecular weight. For example, when the m/z value of the fragment ion is 200, the molecular weight of the fragment ion may be said to be 200 Dalton (g/mol). Among the fragment ion spectrum information, molecular weight information is used to acquire the molecular structure of fragment ions from the spectrum-molecular structure DB 400.

The ionic intensity value is related to the amount of fragment ions generated. A high ionic intensity value means that the number of times pieces are cut is high, and usually, since fragment ions positioned in the center of a compound molecule have high stability and are less likely to be cut into fragment ions, when a final parent molecule candidate structure is derived, fragment ions with high ionic intensity values are positioned in the center, and thus a structure having to break multiple bonds is highly likely to be a candidate structure that does not fit the actual parent molecule structure.

The spectrum preprocessing module 200 acquires mass spectrum peak data (molecular weight, ionic intensity value of each fragment ion) of each of fragment ions constituting the target substance from the LC-MS/MS measuring unit 100, acquires the molecular structure of fragment ions by inputting the data into the spectrum-molecular structure DB, and reconstructs the molecular structures of the acquired fragment ions into fragment ion graph data. The spectrum-molecular structure DB is a database containing known mass spectrum data and corresponding molecular structure data on molecules and known spectrum-molecular structure DBs include NIST Chemistry WebBook, NIST Mass Spectral Library, METLIN, PubChem, ChemSpider, and the like, but the scope of the present invention is not limited to the use of a specific database, and as a database loaded with its own data, any database containing mass spectrum data on a given molecule and corresponding molecular structure data may be applied to the system of the present invention.

The spectrum preprocessing module 200 receives molecular structure data on the fragment ions corresponding to the mass spectrum data on the fragment ions input from the spectrum-molecular structure DB and reconstructs the received data into graph data.

In the present invention, "graph" refers to an expression of a certain molecular structure in the form of a graph, and "graph data" refers to a conversion of the graph into numerical data that is able to be processed by computer operations. Graph data may include nodes that make up the graph, edges that link nodes, node properties, and edge property data, and may be expressed as matrix data such as an adjacency matrix or the like that expresses them, but is limited to a specific data format.

The fragment ion graph, which the molecular structures of fragment ions are reconstructed as graph data according to the present invention, expresses each invariant atom group as one node, expresses atoms that are not invariant atom groups as other nodes linked to the invariant atom group node, and has an edge linking each node (node: this is a node expressing the molecular structure and is different from the "tree node," "parent node," and "child node" described below). The fragment ion graph data may be graph data including variables expressing each node of the fragment ion graph and node attribute data, and edge variables linking the nodes and edge attribute data.

The graph assembly module 300 may receive fragment ion graph data obtained by graphing the fragment ions, assemble fragment ion graphs according to a fragment ion assembly method of the present invention, which will be described below, and calculate assembly intermediate graph data, which is fragment ion combining molecular graph data, and further output the assembly intermediate graph data as a fragment ion binding molecular structure, that is, a candidate structure for the estimated parent molecule. The graph assembly module 300 may include a parent molecular structure determination module (not shown) to determine the final parent molecular structure from the candidate structures through a likelihood evaluation operation described below.

In the present invention, the spectrum preprocessing module 200 and the graph assembly module 300 may each be a separate data operation module, or may be a single computing device or computer device. In this case, each calculation module, calculation device, or computer device may be configured to include a storage device and a calculation device that store, read, and calculate a computer-implemented algorithm for performing graphing of the fragment ions and a graph combining method, which will be described below.

2. Method of acquiring molecular structure of parent molecule

Referring to FIG. 3, the method of acquiring the molecular structure of the parent molecule according to the present invention will be generally described.

(1) Fragment ion candidate structure acquisition step (S100)

First, by performing the following procedures, candidate structures of fragment ions are acquired.

In order to derive the molecular structure of a given target substance (target compound, parent molecule) having the molecular structure that is unknown, LC-MS/MS analysis is first performed on a sample containing the target substance.

In LC-MS/MS analysis, the target substance in the sample, that is, the target compound in the sample, is separated through liquid chromatography (LC), the target compound is input into the MS/MS equipment, fragment ions are generated, and a mass spectrum (MS/MS spectrum) for the generated fragment ions is generated, where the present invention begins a process of estimating the molecular structure of the parent molecule by acquiring mass spectrum data on the fragment ions generated at this time.

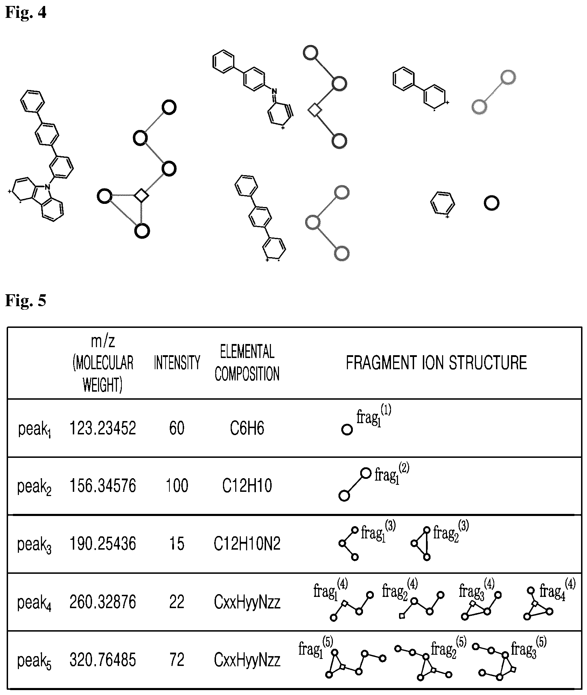

Next, the mass spectra for the acquired fragment ions are input into the spectrum-molecular structure DB 400 to derive a candidate structure of a fragment ion matching a corresponding spectrum. As shown in the example in FIG. 4, since it is difficult to know in advance which fragment ion will correspond to each peak in the mass spectrum, a list of all fragment ions of the same elemental composition is retrieved and assembly is performed for each of them. A number of fragment ions that make up the list of the fragment ions corresponding to each peak in the mass spectrum are referred to as candidate fragments.

(2) Fragment ion graphing step (S200)

Next, this is a step of acquiring fragment ion graph data by performing fragment ion graphing of the molecular structures of the candidate fragments acquired from the database and converting the fragment ion graphs into data that is able to be processed by computer operations. Converting the fragment ion graph into the fragment ion graph data that is able to be processed by computer operations is to express the fragment ion graph as a graph adjacency matrix, node attribute matrix, and edge attribute matrix including graph nodes and their attribute information, edges, and edge attribute information as described below, which is well known to those skilled in the art.

In the present invention, fragment ion candidate structures are referred to as candidate fragments, and when representing them as graph data, the invariant atomic group contained in the fragment ion, which is always detected in a combined form on the MS/MS spectrum, is expressed as a single node. Atoms that do not belong to the invariant atomic group are expressed as one node. The information about each invariant atomic group and atom is expressed as attribute information about each node.

For example, as shown in FIG. 4, benzene is an invariant atomic group that is always detected in its complete form and is treated as one node. However, for carbazole, the central pentagonal structure is sometimes decomposed and detected as a bond of benzene and nitrogen atoms, and thus, the carbazole is not treated as an invariant atomic group, but is expressed as a linkage between a benzene node and a nitrogen node, which are invariant atomic groups.

(3) Fragment ion assembly step (S300)

Next, fragment ion graphs representing candidate structures of fragment ions are assembled to derive candidate structures of the parent molecule.

However, it is difficult to know in advance which fragment ion will correspond to a peak of the MS/MS spectrum. Therefore, all lists of the fragment ions with elemental compositions corresponding to each peak of the spectrum are retrieved from the spectrum-molecular structure DB 400, and then assembly is performed for each of them. When there are N peaks $\{p_1, p_2, ...., p_N\}$ in the mass spectrum, and there are $n_i$ candidate fragment ions that are able to correspond to each peak $P_i$, the total number of fragment ion combinations that have to be be assembled becomes $\prod_{i=1}^{N} n_i$, and the calculation takes a very long time.

Therefore, in the present invention, when any two combinations share most of the fragment ion combinations, it is determined that it would be inefficient to assemble each of them from scratch, and a method of minimizing duplicate path visits by conducting an assembly process in the form of a search tree is applied. During the tree search process, pruning and excluding nodes that may no longer be assembled eliminates the need to visit all nodes branching from that node, thereby drastically shortening the time. This will be described with reference to FIG. 5.

In the present invention, N peaks are listed in order of size, and then a search tree for which a search node is an action of selecting one of the fragment ion candidates is created. A node with depth i corresponds to an action of selecting which fragment ion to assemble at an i-th peak. Applying the principle, the present invention performs tree search in the following order. In this way, the tree search process assigns the fragment ion graphs corresponding to each peak to the corresponding tree node variables and performs a tree search operation.

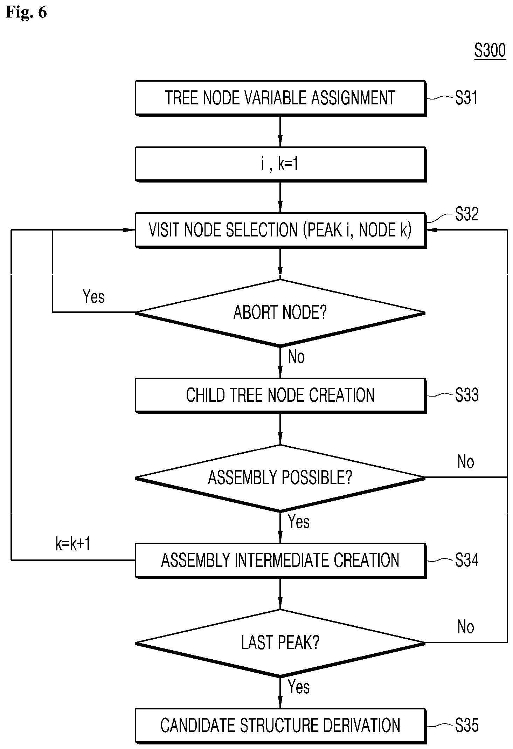

FIG. 5 is a view illustrating the process of selecting bound fragment ions when there are five peaks in the mass spectrum and the number of candidate fragment ions corresponding to peaks is three, four, two, one, and one, respectively. With reference to FIGS. 5 and 6, a specific procedure of tree search operation will be described.

In the example of FIG. 5, three tree nodes are assigned to peak$_1$, and four tree nodes are assigned to peak$_2$. Each tree node variable corresponds to fragment ion graph data of each fragment ion. In the example of FIG. 5, the number of possible combinations of fragment graphs is 3 * 4 * 2 * 1 * 1 = 24 cases, and according to the combining method of the present invention, the number of possible combinations is reduced, saving computational resources.

(a) Tree node assignment step (S31)

In order to perform a tree search operation, fragment ion graphs corresponding to each peak of the mass spectrum are assigned to each tree node variable of the search tree. A root node of the search tree is set to be with an empty graph. Corresponding fragment ion graphs are assigned to each node of each peak, and each fragment ion graph may be expressed as g_frag$_i^{(k)}$ (k represents a peak number and i represents a fragment graph number of the peak).

Fragment ion graphs corresponding to each peak are assigned to tree nodes for each depth of the search tree. For example, a starting node of the search tree is assigned to a root node with an empty graph, and the fragment ion graphs corresponding to peak_b are assigned to tree nodes at depth b.

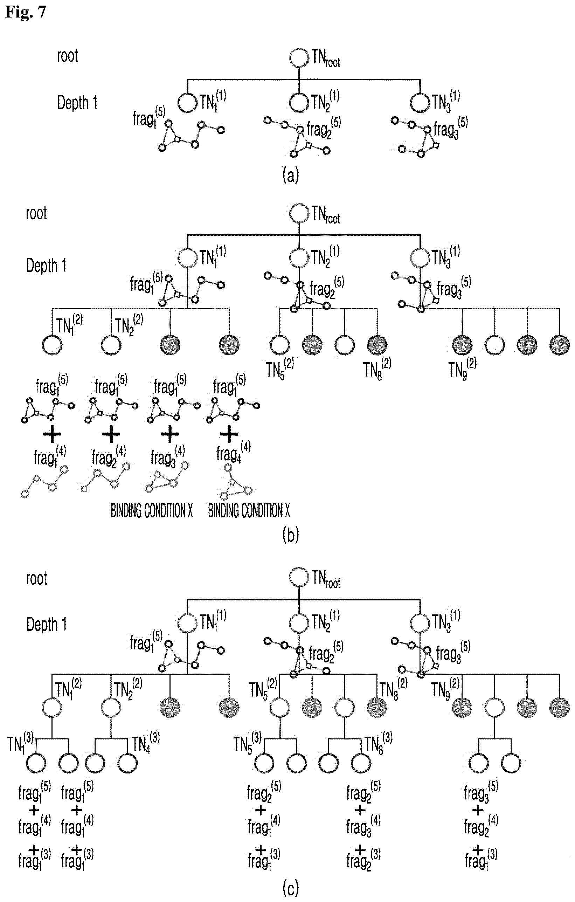

As shown in FIG. 7, in the search tree, the starting node may be TN$_{root}$, and other tree nodes may be expressed as TN$_a^{(b)}$ variables (b is the depth of the search tree, and a is the order of tree nodes at that depth).

(b) Visit node selection step (S32)

This is a step of sequentially selecting and visiting the tree nodes to be visited among the tree nodes. In practice, the step may be implemented with a computer algorithm in which a predetermined tree node selection pointer sequentially points to each of the tree nodes.

Fragment graph assembly procedures to be described below are performed by selecting the first tree node of the first peak, graph assembly procedures to be described below are performed by sequentially selecting all corresponding tree nodes of one peak, and then tree nodes of the next peak are selected. Meanwhile, the selection order of tree nodes is not limited thereto, and it is sufficient to go through the process of selecting all tree nodes of all peaks regardless of their order.

The selection of tree nodes is performed in a procedure to be described below, excluding tree nodes marked as "abort" according to a predetermined condition. The indication of "abort" may be displayed as "abort" for the tree node by changing the value of a certain flag in the tree node variables, and the indication of "abort" does not mean stopping the procedure, but rather means that the graph assembly is not performed for the fragment graph corresponding to the tree node.

The visit node selection step may begin with visiting the first parent node among the top parent nodes. In the subsequent iteration step, when the visit node is marked as "abort", the next node is visited without performing a subsequent procedure.

(c) Child node creation step (S33)

For the visited tree node, a child node corresponding to each of fragment ion graphs of a peak following the peak to which the corresponding tree node corresponds is created. This is a step of generating the number of cases in which each fragment ion candidate that is able to correspond to the following peak is assembled in the graph assembled so far.

(d) Assembly intermediate graph generation step (S34)

This is the step of generating an assembly intermediate graph at each child node by assembling the corresponding fragment ion graph (child node graph) with the assembly intermediate graph of the parent node at each child node. Each tree node may be assigned a corresponding assembly intermediate node variable, and as the child node, the assembly intermediate graph of its parent node and the assembled assembly intermediate graph may be stored in correspondence.

In this case, in the assembly intermediate graph generation step (S34), at each child node, the assembly intermediate graph stored in the assembly intermediate node variable of the parent node and child node graph are assembled, and graph data as the assembly result is stored in the assembly intermediate node variable of the child node to be used for assembly with the child node graph of the next depth. After generating the assembly intermediate graphs for all child nodes, when a corresponding peak is not the last peak, each child node is visited and the process returns to the visit node selection step (S32).

Meanwhile, among the child nodes that satisfy a predetermined condition to be described below, child nodes that are not able to create any assembly intermediate with the parent node are added to the list of abort nodes.

(e) Parent molecule candidate structure derivation step (S35)

This is a step of acquiring a final updated assembly intermediate graph by repeating steps S32 to S34 until a node of depth N corresponding to the last peak of the peak list is created, and acquiring a candidate molecular structure of the parent molecule (target substance) therefrom.

For the example of FIG. 5, the above-described procedures S31 to S35 will be described with reference to FIG. 7 as an example. In the example of FIG. 5, since there are five peaks from $p_1$ to $p_5$ and the fragment ions corresponding to each peak are one, one, two, four, and three, respectively, the number of graph combinations to be considered in the normal method is 1 * 1 * 2 * 4 * 3 = 24 types. However, according to the abort node pruning method applied in the present invention, the number of combinations is reduced, thereby increasing computational efficiency.

The assembly order of each peak may be arbitrarily selected, and in FIG. 7, a case of assembly in the order $p_5$ -> $p_4$ -> $p_3$ -> $p_2$ -> $p_1$ will be described. $TN_a{}^b$ is assigned to each tree node variable in the search tree, where a represents a node serial number and b represents a tree depth. For example, $TN_2{}^{(1)}$ refers to a second node at Depth 1, and $TN_{12}{}^{(2)}$ refers to a 12-th node at Depth 2. Likewise, the assembly intermediate graph corresponding to each tree node $TN_a{}^{(b)}$ is expressed as $G_a{}^{(b)}$.

(a) in FIG. 7 shows an initial start of a tree search, that is, a step in which a root node is selected as a visit node (S32).

In the next step, at Depth 1, child nodes to which fragment ion graphs $frag_{1\ to\ 3}{}^{(5)}$ corresponding to $peak_5$ are assigned are generated (S33) as child nodes of the visit node. As for the child nodes, in the example of FIG. 5, since three fragment ion graphs correspond to $peak_5$, three child nodes are created. In the next step, each child node is assembled with the root node to generate an assembly intermediate graph (S34). Since the root node has no corresponding peak or fragment ion, at Depth 1, the assembly intermediate graph generated from the three child nodes is the same as each fragment ion graph at $peak_5$. That is, at Depth 1, the tree nodes $TN_{1\ to\ 3}{}^{(1)}$ are created as child nodes of the root node, and the fragment ion graphs $frag_{1\ to\ 3}{}^{(5)}$ are assigned assembly intermediates $G_{1\ to\ 3}{}^{(1)}$ corresponding to the tree nodes $TN_{1\ to\ 3}{}^{(1)}$, respectively (a fragment ion graph is expressed as $frag_a{}^{(b)}$, where b is a peak number to which the fragment ion corresponds, and a is the fragment ion graph number of the corresponding peak.)

Since the peak in the procedure shown in (a) in FIG. 7 is not the final peak, as shown in (b) in FIG. 7, by returning to the visit node selection step, the tree nodes corresponding to the generated $peak_5$ are selected as visit nodes (S32), and for each tree node of $peak_5$, child nodes to which the fragment ion graphs corresponding to $peak_4$ are assigned are created (S33). Since four fragment ions correspond to $peak_4$, four child nodes are assigned to each of the nodes corresponding to

peak$_5$. Now, at each of the child nodes (a total of 12), the assembly intermediate graph of the parent node and the fragment ion graph corresponding to the child node are combined to generate an assembly intermediate graph corresponding to the child node in this step (S34). An assembly intermediate $G_1^{(2)}$ corresponding to $TN_1^{(2)}$ is $frag_1^{(5)}$ + $frag_1^{(4)}$, and an assembly intermediate $G_2^{(2)}$ corresponding to $TN_2^{(2)}$ is $frag_1^{(5)}$ + $frag_2^{(4)}$.

When the molecular weight in the assembly intermediate graph resulting from such a combination becomes greater than the molecular weight of the parent molecule before the target substance is input into a mass spectrometer, the combination does not seem to satisfy the binding condition, and thus the node is set as an abort node. In FIG. 7, the case where since the assembly intermediate graphs with the parent node assembly intermediates assembled in $TN_3^{(2)}$, $TN_4^{(2)}$, $TN_6^{(2)}$, $TN_8^{(2)}$, $TN_9^{(2)}$, and $TN_{11}^{(2)}$ do not satisfy the predetermined combination to be described below, $TN_3^{(2)}$, $TN_4^{(2)}$, $TN_6^{(2)}$, $TN_8^{(2)}$, $TN_9^{(2)}$, and $TN_{11}^{(2)}$ are set as abort nodes is shown.

Since the last peak has not yet been reached, by returning to the visit node selection step (S32), as shown in (c) in FIG. 7, for each tree node of peak$_2$, the tree nodes of peak$_3$ are created as child nodes (S34), and an assembly intermediate graph corresponding to each tree node of peak$_2$ is generated. In this case, even when $TN_3^{(2)}$, $TN_4^{(2)}$, $TN_6^{(2)}$, $TN_8^{(2)}$, $TN_9^{(2)}$, and $TN_{11}^{(2)}$, which have been previously set as abort nodes, are visited, the child node creation and assembly intermediate creation steps are not performed, so that the amount of computation may be reduced. In this way, for all nodes $TNa^{(3)}$ at Depth 3, the visit, child node creation, and assembly intermediate creation processes are performed, and then the visit node selection step is performed again at the next depth. This process is repeated until the last peak is reached.

(f) Parent molecular structure determination step (S36)

Next, an optimal candidate molecular structure is selected from among the candidate molecular structures that are the final assembly intermediates assembled while the last peak is reached, and is determined as a final parent molecular structure. The final parent molecular structure may be determined through likelihood evaluation of candidate molecular structures.

When it is assumed that a derived candidate molecular structure is $\zeta$ and the fragment ion mass spectrum applied to derive the candidate molecular structure is $X = x_0, x_1, ..., x_i$ ($x_k$ is a peak group consisting of consecutive deprotonation peaks, that is, a set of consecutive peaks with a difference of one hydrogen), in this case, the probability of the occurrence of the peak group X by the candidate molecular structure $\zeta$ may be expressed as the product of the probabilities of occurrences of individual peaks in the set X occurs by the candidate molecular structure $\zeta$, as shown in Equation 1 below, and the likelihood evaluation is to find a structure that maximizes a likelihood expressed by Equation 1.

(Equation 1)

$$p(\zeta \mid X) \sim \Pi_k p(\zeta \mid x_k)$$

When, it is assumed that, for each peak $x_i^{(k)} = (m_i^{(k)}, ri^{(k)})$ with m/z $m_i^{(k)}$ and normalized intensity $r_i^{(k)}$ in the peak group $x_k$, a corresponding structure among the partial structures of $\zeta$ is $S_{m_i^{(k)}}$, the likelihood for the peak group may be expressed as Equation 2 below again.

(Equation 2)

$$p(\zeta \mid x_k) = \Pi_i p(S_{m_i^{(k)}} \mid r_i^{(k)})$$

($m_i^{(k)}$ and $r_i^{(k)}$ is the m/z and the normalized intensity of the peak group $x_k$, respectively)

Here, in order to generate $S_{m_i^{(k)}}$, from $\zeta$, fragmentation has to be performed $a_i^{(k)}$ times and deprotonation has to be performed $b_i^{(k)}$ times, and assuming that each probability is expressed as constants independent of the structure, $p_j$ (probability of breaking a certain binding) and $p_j$ (probability of one additional hydrogen being removed from a substructure), maximizing the likelihood $p(S_{m_i^{(k)}} \mid s_i^{(k)})$ ) for an individual peak is equivalent to minimizing the crossentropy in Equation 3 below.

(Equation 3)

$$crossentropy = -r_i^{(k)}\log(p_f^{a_i^{(k)}}p_d^{b_i^{(k)}})$$

(log$p_j$ and log$p_d$ are each a hyperparameter and are values selected from the above-mentioned spectrum-molecular structure database, a is the number of bindings that have to be broken to generate a fragment ion graph S in the final result graph $\zeta$, and b is the number of hydrogens additionally removed from the fragment ion)

Combining Equations 1 to 3, a fitness score of each candidate structure may be defined as Equation 4 below.

(Equation 4)

$$score(\zeta, X)=-\sum_k\sum_i r_i^{(k)}(a_i^{(k)}\log p_f + b_i^{(k)}\log p_d)$$

(log$p_j$ and log$p_d$ are each a hyperparameter and are values selected from the above-mentioned spectrum-molecular structure database, a is the number of bindings that have to be broken to generate the fragment ion graph S in the final result graph $\zeta$, and b is the number of hydrogens additionally removed from the fragment ion)

In this way, the fitness for each candidate structure is calculated through calculation according to the equations, and according to the calculated fitness, the final candidate structure of the target substance is calculated from the final assembly intermediate graph obtained by assembling the fragment ion graphs.

(4) Detailed description of the assembly intermediate graph generation step (S34)

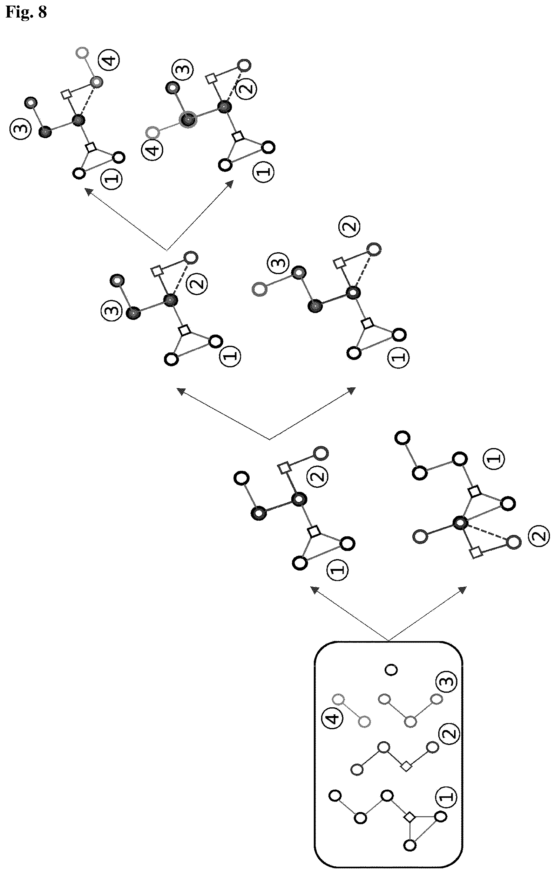

In the assembly intermediate graph generation step, the algorithm for generating the assembly intermediate graph will be described in more detail with reference to FIGS. 8 to 11.

Since each peak in the MS/MS spectrum represents the fragment ion obtained by decomposing the parent molecule using different methods, there is overlap between the molecular structures of the detected candidate fragments. Therefore, when fragment ion graphs are combined, overlap has to be allowed and the number of all possible cases of assembling the given graphs according to the elemental composition of the parent molecule has to be extracted and assembled.

FIG. 8 shows an example in which, when assembling is performed in the order of ① → ② → ③ → ④, only two cases are picked up in each step, schematically illustrating a form in which assembly is possible by allowing overlap.

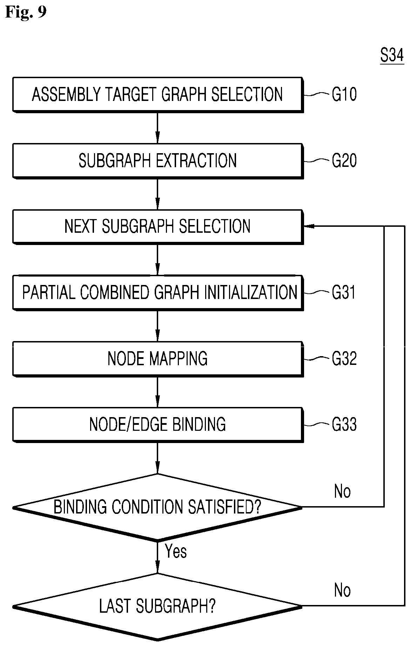

FIG. 9 shows a specific algorithm for the assembly intermediate generation procedure (S34).

(a) Assembly target graph selection step (G10)

Two graphs to be assembled from the fragment ion graphs expressing candidate structures of the fragment ion are selected. Selection of the graph to be assembled is repeated until all fragment ion graphs are selected as assembly targets and assembly is completed.

This step is a step of selecting the assembly intermediate graph of the parent node and the fragment ion graph of the child node as target fragment ion graph data to be assembled and an input assembly fragment ion graph to be assembled, respectively, after creating the child tree nodes at the visit node in the previous S33.

(b) Subgraph extraction step (G20)

A step of selecting and assembling the first fragment ion graph to be assembled is referred to as step t, and a step of selecting and assembling the second graph to be assembled is referred to as step t+1.

Among the graphs to be assembled, the fragment ion graph to be assembled is referred as a target graph (current graph), and the target graph at step t is called $g_c^{(t)}$ (current graph at step t). The graph to be assembled is referred to as an input graph (incoming graph) and is expressed as $g_i$ (incoming graph). A set of all resulting graphs where the two graphs are assembled is referred to as $G^{(t+1)}$,

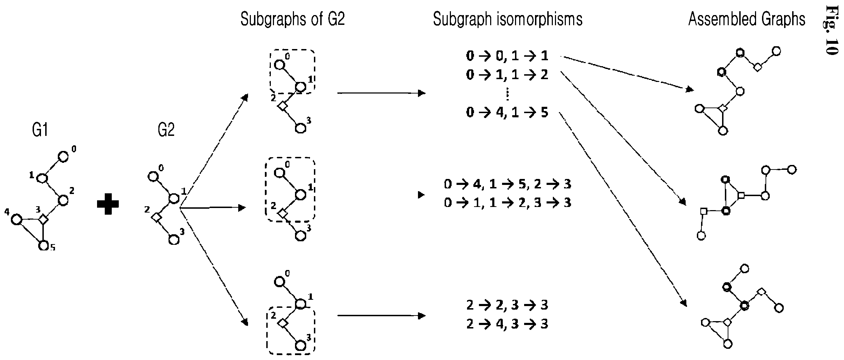

FIG. 10 shows a case where G1 is selected as the target graph $g_c$ and G2 is selected as the input graph $g_i$. G1 includes node $n_i$ (i=0 to 5), and G2 includes node $n_k$ (k = 0 to 3).

This is a step of extracting all subgraphs Si of the graph to be assembled, that is, $g_i$ of the input graph (incoming graph). FIG. 10 shows that three subgraphs are extracted, with nodes (0, 1), (0, 1, 2), and (2, 3) of G2 as nodes. The algorithm for extracting a subgraph from a graph is a known algorithm.

(c) Subgraph assembly step (G30)

Next, a subgraph assembly step is performed in which all subgraphs Si of the input graph $g_i$ are sequentially assembled into $g_c$.

① Partial graph initialization step (G31)

The assembly of the subgraph begins by setting the target graph (current graph) to $g_c^{(t)}$, an incoming graph to be assembled to $g_i$, and a resulting graph set $G^{(t+1)}$ to an empty set.

② Node mapping step (G32)

Next, for each subgraph Si $(\varepsilon g_i)$ of the incoming graph $g_i$, isomorphic graph identification (subgraph isomorphism) and node mapping with the target graph $g_c^{(t)}$ are performed.

③ Node/Edge binding step (G33)

When it is assumed that mapping of all nodes M, the subgraph isomorphic graph identification of Si and $g_c^{(t)}$ is $M_k=\{(v_i,v_c)|v_i\varepsilon S_i,v_c\varepsilon g_c^{(t)}\}$, and a complement graph of subgraph Si is Si$^c$, for each and every node mapping $M_k(\varepsilon M)$, ⓐ a procedure of substituting $g_c^{(t)}$ into $g_c^{(t+1)}$ and adding all nodes and edges of Si$^c$ to $g_c^{(t+1)}$, ⓑ a procedure of adding all edges of $g_i$ that do not belong to Si and Si$^c$ to $g_c^{(t+1)}$ and ⓒ a procedure of adding $g_c^{(t+1)}$ to $G^{(t+1)}$ are performed. In this case, the sum of the molecular weight of $g_c^{(t)}$ and the molecular weight of Si$^c$ has to be equal to or smaller than the molecular weight of the parent molecule. When the sum of the molecular weight of $g_c^{(t)}$ and the molecular weight of Si$^c$ exceeds the molecular weight of the parent molecule, a determination of inability to bind is made.

The above procedures ② and ③ are performed for all subgraphs Si of $g_i$.

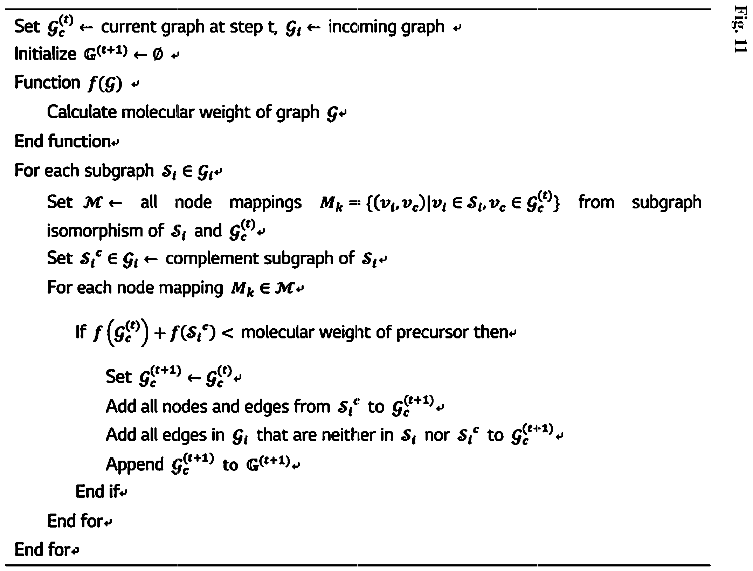

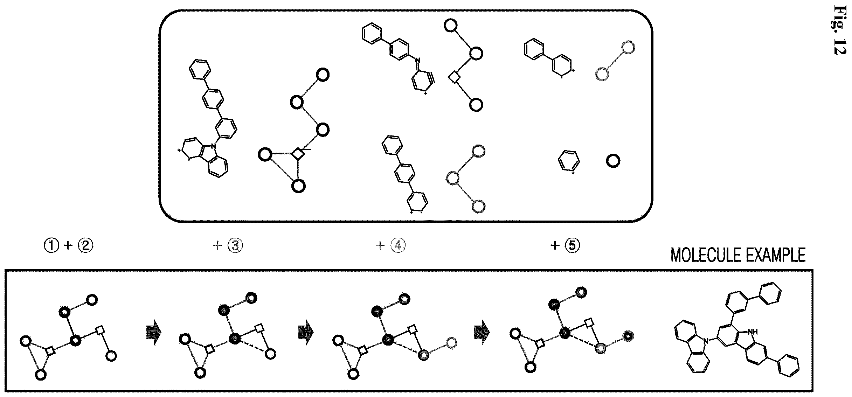

An example of pseudocode that performs the above-described procedures is shown in FIG. 11, and an example of a resulting graph of binding through the procedures is shown in FIG. 12.

Names of respective symbols in drawings used in the present invention are as follows.

| 100 | LC-MS/MS MEASURING UNIT | 200 | SPECTRUM PREPROCESSING MODULE |
|-----|-------------------------|-----|-------------------------------|
| 300 | GRAPH ASSEMBLY MODULE | 400 | SPECTRUM-MOLECULAR STRUCTURE DB |

**Claims**

1. A method of acquiring a molecular structure of a target substance, the method comprising:

   a fragment ion candidate structure derivation step of deriving candidate structures of fragment ions by inputting mass spectra for fragment ions acquired from LC-MS/MS analysis of the target substance into a predetermined spectrum-molecular structure database;
   a fragment ion graphing step of converting the candidate structures of the derived fragment ions into fragment ion graph data;
   a fragment ion graph assembly step of assembling fragment ion candidate structures by using the fragment ion graph data to acquire candidate molecular structures of the target substance; and
   a target substance molecular structure determination step of determining a target substance molecular structure from the candidate molecular structures of the target substance in the fragment ion graph assembly step.

2. The method of claim 1, wherein in the fragment ion graphing step, invariable atomic groups included in the fragment ion and linking atoms linked to the invariable atomic groups are expressed as nodes, and information on the atomic group or linking atom represented by each of the nodes is expressed as attribute information about each node.

3. The method of claim 2, wherein the fragment ion graph assembly step comprises:

   a tree node assignment step (S31) of assigning fragment ion graphs corresponding to each peak of the mass spectrum to a tree node variable $TN_a^{(b)}$ wherein b is a depth of a search tree, and a is an order of tree nodes at the depth in order to perform a tree search operation;

a visit node selection step (S32) of sequentially selecting and visiting tree nodes of a first depth, excluding tree nodes set as abort nodes;

a child tree node creation step (S33) of setting the visited tree node $TN_a^{(b)}$ as a parent node and linking all tree nodes $TN_a^{(b+1)}$ corresponding to a following peak as child nodes;

an assembly intermediate creation step (S34) of creating assembly intermediates in the child nodes by sequentially assembling the fragment ion graphs assigned to the child nodes $TN_a^{(b+1)}$ with assembly intermediate graphs assigned to intermediate node variables of the parent node $TN_a^{(b)}$, assigning an assembly intermediate to an assembly intermediate node variable corresponding to a corresponding child node, and setting the corresponding child node as the abort node when a predetermined assembly intermediate binding condition is not satisfied; and

acquiring, as candidate molecular structures of the target substance, final assembly intermediates created by repeating the visit node selection step to the assembly intermediate creation step until there are no more child tree nodes to be created.

4. The method of claim 3, wherein the assembly intermediate creation step comprises:

an assembly target fragment ion selection step of selecting the assembly intermediate graph of the parent node and the fragment ion graph of the child node as target fragment ion graph data to be assembled and an input assembly fragment ion graph to be assembled, respectively;

a subgraph data extraction step of extracting all subgraphs from the input fragment ion graph data; and

a subgraph combination step of sequentially combining all the subgraphs into the target fragment ion graph.

5. The method of claim 4, wherein in the assembly intermediate creation step, at a time of combining the subgraphs, when a molecular weight of the molecule corresponding to the combined graph exceeds the molecular weight of the parent molecule, it is determined that the assembly intermediate binding condition is not satisfied, and the corresponding child node is set as the abort node.

6. The method of claim 4, wherein in the target substance molecular structure determination step, fitness scores of candidate molecular structures of the target substance are calculated and the molecular structure of the target substance from the calculated fitness scores is determined.

7. A system of acquiring a molecular structure of a target substance, the system comprising:

an LC-MS/MS measuring unit configured to separate the target substance into fragment ions and generate mass spectra for the fragment ions;

a spectrum preprocessing module configured to acquire mass spectrum peak data of fragment ions from the mass spectra of the fragment ions, acquire the molecular structures of fragment ions from a spectrum-molecular structure database using the mass spectrum peak data, and perform fragment ion graphing on the molecular structures of fragment ions to produce fragment ion graph data;

a graph assembly module configured to derive candidate molecular structures of the target substance by receiving the fragment ion graph data and assembling fragment ion graphs; and

a spectrum-molecular structure database configured to output molecular structures of the fragment ions by receiving mass spectral peak data on the fragment ions.

8. The system of claim 7, wherein the spectrum preprocessing module performs fragment ion graphing on a fragment ion molecular structure by expressing invariable atomic groups included in the fragment ion and the linking atoms linked to the invariable atomic groups as nodes and expressing the information about the atomic group or linking atom represented by each node as attribute information about each node.

9. The system of claim 8, wherein the graph assembly module derives candidate molecular structures of the target substance by assembling fragment ion graphs corresponding to each peak of the fragment ion spectrum, and when a molecular weight of a molecule corresponding to the combined graph is not smaller than a molecular weight of a precursor ion, it is determined that an assembly intermediate binding condition is not satisfied, and assembly after the corresponding fragment graph is not performed any further.

10. A device of calculating a molecular structure of a target substance, the device comprising:

a spectrum preprocessing module configured to receive a fragment ion spectrum of the target substance from an LC-MS/MS measuring instrument and calculate fragment ion graph data by graphing molecular structures of fragment ions; and

a graph assembly module configured to derive candidate molecular structures of the target substance by receiving the fragment ion graph data and assembling fragment ion graphs.

11. The device of claim 10, wherein the spectrum preprocessing module performs fragment ion graphing on a fragment ion molecular structure by expressing invariable atomic groups included in the fragment ion and the linking atoms linked to the invariable atomic groups as nodes and expressing the information about the atomic group or linking atom represented by each node as attribute information about each node.

**Fig. 1**

**Fig. 2**

MS/MS Spectrum

**Fig. 3**

**Fig. 4**

**Fig. 5**

| | m/z (MOLECULAR WEIGHT) | INTENSITY | ELEMENTAL COMPOSITION | FRAGMENT ION STRUCTURE |
|---|---|---|---|---|
| $peak_1$ | 123.23452 | 60 | C6H6 | $frag_1^{(1)}$ |
| $peak_2$ | 156.34576 | 100 | C12H10 | $frag_1^{(2)}$ |
| $peak_3$ | 190.25436 | 15 | C12H10N2 | $frag_1^{(3)}$  $frag_2^{(3)}$ |
| $peak_4$ | 260.32876 | 22 | CxxHyyNzz | $frag_1^{(4)}$  $frag_2^{(4)}$  $frag_3^{(4)}$  $frag_4^{(4)}$ |
| $peak_5$ | 320.76485 | 72 | CxxHyyNzz | $frag_1^{(5)}$  $frag_2^{(5)}$  $frag_3^{(5)}$ |

**Fig. 6**

S300

```
           ┌─────────────────────────────────────┐
           │   TREE NODE VARIABLE ASSIGNMENT      │──S31
           └─────────────────────────────────────┘
                            │
                            ▼
           ┌─────────────────────────────────────┐
           │              i , k=1                 │
           └─────────────────────────────────────┘
                            │
                            ▼
           ┌─────────────────────────────────────┐
           │  VISIT NODE SELECTION (PEAK i, NODE k) │──S32
           └─────────────────────────────────────┘
                            │
                            ▼
  Yes              ╱◇ ABORT NODE? ◇╲
 ◄─────────────────◇               ◇
                    ╲               ╱
                       │ No
                       ▼
           ┌─────────────────────────────────────┐
           │      CHILD TREE NODE CREATION       │──S33
           └─────────────────────────────────────┘
                       │
                       ▼                              No
                ◇ ASSEMBLY POSSIBLE? ◇ ────────────►
                       │ Yes
         k=k+1         ▼
 ◄─────────┌─────────────────────────────────────┐
           │   ASSEMBLY INTERMEDIATE CREATION     │──S34
           └─────────────────────────────────────┘
                       │
                       ▼                              No
                    ◇ LAST PEAK? ◇ ───────────────►
                       │ Yes
                       ▼
           ┌─────────────────────────────────────┐
           │    CANDIDATE STRUCTURE DERIVATION    │──S35
           └─────────────────────────────────────┘
```

**Fig. 7**

(a)

(b)

(c)

Fig. 8

**Fig. 9**

S34

```
┌──────────────────────────────────┐
│  ASSEMBLY TARGET GRAPH SELECTION │──G10
└──────────────────────────────────┘
                 │
                 ▼
┌──────────────────────────────────┐
│       SUBGRAPH EXTRACTION        │──G20
└──────────────────────────────────┘
                 │
                 ▼
┌──────────────────────────────────┐
│      NEXT SUBGRAPH SELECTION     │◄────────┐
└──────────────────────────────────┘         │
                 │                            │
                 ▼                            │
┌──────────────────────────────────┐         │
│ PARTIAL COMBINED GRAPH INITIALIZATION│──G31 │
└──────────────────────────────────┘         │
                 │                            │
                 ▼                            │
┌──────────────────────────────────┐         │
│          NODE MAPPING            │──G32     │
└──────────────────────────────────┘         │
                 │                            │
                 ▼                            │
┌──────────────────────────────────┐         │
│        NODE/EDGE BINDING         │──G33     │
└──────────────────────────────────┘         │
                 │                            │
                 ▼                      No    │
         ◇ BINDING CONDITION SATISFIED? ◇─────┤
                 │ Yes                        │
                 ▼                      No    │
              ◇ LAST SUBGRAPH? ◇─────────────┘
```

Fig. 10

Fig. 11

EP 4 521 413 A1

Set $\mathcal{G}_c^{(t)} \leftarrow$ current graph at step t, $\mathcal{G}_i \leftarrow$ incoming graph

Initialize $\mathbb{G}^{(t+1)} \leftarrow \emptyset$

Function $f(\mathcal{G})$

    Calculate molecular weight of graph $\mathcal{G}$

End function

For each subgraph $S_i \in \mathcal{G}_i$

    Set $\mathcal{M} \leftarrow$ all node mappings $M_k = \{(v_i, v_c) | v_i \in S_i, v_c \in \mathcal{G}_c^{(t)}\}$ from subgraph isomorphism of $S_i$ and $\mathcal{G}_c^{(t)}$

    Set $S_i^c \in \mathcal{G}_i \leftarrow$ complement subgraph of $S_i$

    For each node mapping $M_k \in \mathcal{M}$

        If $f\left(\mathcal{G}_c^{(t)}\right) + f(S_i^c) <$ molecular weight of precursor then

            Set $\mathcal{G}_c^{(t+1)} \leftarrow \mathcal{G}_c^{(t)}$

            Add all nodes and edges from $S_i^c$ to $\mathcal{G}_c^{(t+1)}$

            Add all edges in $\mathcal{G}_i$ that are neither in $S_i$ nor $S_i^c$ to $\mathcal{G}_c^{(t+1)}$

            Append $\mathcal{G}_c^{(t+1)}$ to $\mathbb{G}^{(t+1)}$

        End if

    End for

End for

**Fig. 12**

## INTERNATIONAL SEARCH REPORT

International application No.

**PCT/KR2024/095643**

| A. | CLASSIFICATION OF SUBJECT MATTER |
|---|---|

**G16C 20/70**(2019.01)i; **G16C 20/50**(2019.01)i; **G16C 20/40**(2019.01)i; **G16C 20/20**(2019.01)i; **G16C 20/30**(2019.01)i; **G16C 20/80**(2019.01)i; **G16C 20/90**(2019.01)i

According to International Patent Classification (IPC) or to both national classification and IPC

| B. | FIELDS SEARCHED |
|---|---|

Minimum documentation searched (classification system followed by classification symbols)

G16C 20/70(2019.01); A61B 5/055(2006.01); G01N 27/62(2006.01); G01N 27/626(2021.01); G01N 30/72(2006.01); G01N 33/50(2006.01); G06F 19/00(2011.01); G16C 20/30(2019.01); H01J 49/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 액체 크로마토그래피-질량분석법(liquid chromatography-tandem mass spectrometry, LC-MS/MS), 질량 스펙트럼(mass spectrum), 조각 이온(fragment-ion), 분자구조(molecular structure), 탐색 트리(search tree), 노드(node)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | US 2019-0172695 A1 (JEOL LTD.) 06 June 2019 (2019-06-06)<br>See claim 7; and paragraphs [0007]-[0009] and [0032]-[0077]. | 1-11 |
| Y | CN 114166925 A (XI'AN ELECTRONIC SCIENCE AND TECHNOLOGY UNIVERSITY) 11 March 2022 (2022-03-11) | 1-11 |
| A | US 2019-0294756 A1 (THERMO FINNIGAN LLC et al.) 26 September 2019 (2019-09-26)<br>See entire document. | 1-11 |
| A | US 2018-0011990 A1 (UNIVERSITY OF KENTUCKY RESEARCH FOUNDATION) 11 January 2018 (2018-01-11)<br>See entire document. | 1-11 |

| ☑ Further documents are listed in the continuation of Box C. | ☑ See patent family annex. |
|---|---|

| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
|---|---|

| Date of the actual completion of the international search | Date of mailing of the international search report |
|---|---|
| **17 July 2024** | **18 July 2024** |

| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
|---|---|
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/095643**

**C.     DOCUMENTS CONSIDERED TO BE RELEVANT**

| Category* | Citation of document, with indication, where appropriate, of the relevant passages | Relevant to claim No. |
|---|---|---|
| A | CN 113495094 A (CHINA TELECOM CORPORATION LIMITED) 12 October 2021 (2021-10-12)<br>See entire document. | 1-11 |

Form PCT/ISA/210 (second sheet) (July 2022)

**INTERNATIONAL SEARCH REPORT**
**Information on patent family members**

International application No.

**PCT/KR2024/095643**

| Patent document cited in search report | | | Publication date (day/month/year) | Patent family member(s) | | | Publication date (day/month/year) |
|---|---|---|---|---|---|---|---|
| US | 2019-0172695 | A1 | 06 June 2019 | EP | 3496130 | A1 | 12 June 2019 |
| | | | | JP | 2019-100891 | A | 24 June 2019 |
| | | | | JP | 6994921 | B2 | 14 January 2022 |
| | | | | US | 10804086 | B2 | 13 October 2020 |
| CN | 114166925 | A | 11 March 2022 | CN | 114166925 | B | 26 March 2024 |
| US | 2019-0294756 | A1 | 26 September 2019 | EP | 3544016 | A2 | 25 September 2019 |
| | | | | US | 10878944 | B2 | 29 December 2020 |
| US | 2018-0011990 | A1 | 11 January 2018 | US | 10607723 | B2 | 31 March 2020 |
| CN | 113495094 | A | 12 October 2021 | CN | 113495094 | B | 25 July 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019100891 A

**Non-patent literature cited in the description**

- **ORGANIC MATTER** ; **DANIAL PETRAS**. High-Resolution Liquid Chromatography Tandem Mass Spectrometry Enables Large Scale Molecular Characterization of Dissolved. *METHODS*, 12 December 2017